# EUROPEAN PATENT SPECIFICATION

(11) **EP 0 267 684 B1**
(45) Date of publication and mention of the grant of the patent: **20.07.1994**
(21) Application number: 87308861.1
(22) Date of filing: 06.10.1987
(51) Int. Cl.: A61K 9/06, A61K 37/66

(54) **Human leukocyte interferon composition and skin treatment**
Zubereitung und Behandlung der Haut mit Human-Leukocyten-Interferon
Composition d'interféron leucocytaire humain et traitement de la peau

(30) Priority: 10.11.1986 US 928450
(43) Date of publication of application: 18.05.1988
(73) Proprietor: Viragen, Inc., Hialeah Florida 33016 (US)
(72) Inventor: Munch, David C., Miami Lakes Florida 33014 (US)
(74) Representative: Wilson, Nicholas Martin

(56) References cited:
- EP-A- 0 077 063
- WO-A-83/01198
- US-A- 3 551 554
- US-A- 4 435 384
- US-A- 4 605 555
- Chemical Abstracts, vol. 82, no. 23, June 9, 1975, pp. 25,26, abstract no. 149308g Columbus, Ohio, US, M. Kunze

## Description

This invention relates generally to a topical composition containing human leukocyte interferon and a method of skin treatment using such a composition.

Clinically administered interferon has been demonstrated to possess antitumor activity. H. Strander, K. Cantell, P.A. Jakobsson, U. Nilsonne and G. Soderberg successfully treated patients with osteosarcoma by inducing regression and prolonging remission in patients treated with interferon as described in Exogenous Interferon Therapy of Osteogenic Sarcoma, Act. Orthop. Scand. 45:958-967, 1974. Subsequently, H. Strander demonstrated the efficacy of interferon in the treatment of children with laryngeal papilloma and this work was described in Interferon: Antineoplastic Drugs, Blut 35:277-288, 1977. Interferon therapy has also been used with some benefit in patients with leukemia (J. Loten and L. Sachs, Genetic Dissociation of Different Cellular Effects of Interferon on Myeloid Leukemic Cells, Int. J. Cancer 22:214-220, 1978); non-Hodgkin lymphoma (T.C. Merigan, K. Sikoran, J.G. Breeden, R. Levy and S.A. Rosenbury, Preliminary Observations of the Effect of Human Leukocyte Interferon on Non-Hodgkin's Lymphoma, N. Eng. J. Med. 299:1449-1453, 1978); breast cancer (J.U. Gutterman, G.R. Blumenschein and R. Alexanain et al., Leukocyte Interferon Induced Tumor Regression in Human Metastatic Breast Cancer, Multiple Myeloma, and Maglignant Lymphoma, Ann. Intern. Med. 96:549-556, 1982 and E.C. Borden, J.F. Holland and T.L. Dao et al., Leukocyte Derived Interferon in Human Breast Carcinoma, Ann. Intern. Med. 97:1-6, 1982); and in treatment of renal cell carcinoma (J.R. Quesada, D.A. Swanson, A. Trindade and J.U. Gutterman, Renal Cell Carcinoma: Antitumor Effects of Leukocyte Interferon, Cancer Research 43:940-947, 1983). In addition, less well controlled studies have treated patients with other forms of cancer including lung carcinoma, colon and melanoma. The results of trials conducted utilizing interferon as a cancer treatment have shown some promise. However, treatment of any type of cancer with interferon is still in the investigational stage.

Herpes simplex virus type 1 causes mucocutaneous lesions around the mouth. The type 2 virus, which has been shown to be venereal transmitted, causes lesions in the genital area. A wide variety of treatments including topical, subcutaneous and parenteral have been proposed. For example, acyclovir has been promoted as an antiviral drug active against herpes viruses when used in the form of an ointment. An additional topical composition including transfer factor is described in United States Patent No. 4,435,384 of herpes simplex, blemishes, acne, condyloma and other skin lesions. Topical application is desirable as it can be done without a physician visit. It also permits application of the active ingredient directly to the lesion and the surrounding area and minimizes exposure of the patient to foreign protein which can be a problem with parenteral injections.

Accordingly, it remains desirable to provide an improved topical composition that is effective in the treatment of skin lesions, such as herpes simplex, venereal warts and condyloma.

Generally speaking, in accordance with the invention, a topical composition for dermatological treatment comprises an ointment-type vehicle, including at least an effective amount of human leukocyte interferon dispersed therein and present in an amount of up to 150,000 International Reference Units of Interferon Per Gram of composition, and from an effective amount up to about 15 weight per cent of an enhancing penetrant based on the total weight of the composition. The topical composition contains human leukocyte interferon in a non-toxic vehicle including an enhancing penetrant dispersed therein. In addition, a softening agent may be included.

A typical composition in accordance with the invention includes between 50,000 and 150,000 International Units of human leukocyte interferon per gram of total composition, and up to 15 per cent by weight of an enhancing penetrant, such as dimethyl sulfoxide (DMSO) or low molecular weight dextran. The preferred composition also contains up to 0.5% by weight of a softening agent, such as carboxymethyl cellulose.

The skin lesions are treated by applying the topical composition to the infected area between three and five times per day for between 7 to 10 days with a total application of between 500,000 and 1,500,000 Units of interferon. Therefore, the invention also includes a method of improving the cosmetic appearance of the skin comprising applying to the skin a topical composition in accordance with the invention.

A further object of the invention is to provide an improved topical composition for treatment of skin lesions such as herpes simplex, venereal warts and condylomata. Accordingly, the invention further includes use as a cosmetic product of the topical composition in accordance with the invention.

Yet another object of the invention is to provide an improved topical composition for skin treatment containing human leukocyte interferon, an enhancing penetrant and a softening agent.

The invention accordingly comprises the several steps and the relation of one or more of such steps with respect to each of the others, and the composition possessing the features, properties, and the relation of constituents, which are exemplified in the following detailed disclosure, and the scope of the invention will be indicated in the claims.

The interferon containing topical composition prepared in accordance with the invention contains human leukocyte interferon in a gentle vehicle. The vehicle is preferably a non-toxic carrier and may be an unscented moisturizing formula of the type generally used for dry skin care. The preferred composition includes an enhancing penetrant, such as dimethyl sulfoxide or low molecular weight dextran, for increasing penetration of the human leukocyte interferon into the skin at the site of the lesion. In addition, the preferred composition may include a softening agent such as carboxymethyl cellulose.

Human leukocyte interferon is derived from buffy coats that are rich in leukocytes. Human leukocyte interferon is a protein produced by intact animal cells when infected with viruses. The interferon acts to inhibit viral reproduction and to induce resistance in host cells. Prior to use, the buffy coats used to produce the interferon are screened and must be found free of hepatitis B virus antigen and HTLV-III virus antigen.

The following procedure for producing human leukocyte interferon based on the Cantell method is set forth by way of example. It is set forth for convenience and purposes of illustration only.
1. Buffy coats less than 28 hours old that have been carefully maintained at a temperature between 2° and 10°C are used to produce human leukocyte interferon.
2. The leukocytes are purified by continuous/bucket type centrifugation and hemolysis of the buffy coats in an IEC Chemical Centrifuge or equivalent. Following purification, a sample is obtained in order to determine cell count and viability.
3. A leukocyte culture is prepared in accordance with the following parameters:
   a. Basic culture medium - MEM or equivalent, tricine and bicarbonate buffered
   b. Protein supplement - Human Agamma serum to a protein concentration of between 0.5 and 3.0 mg/ml.
   c. Leukocyte content - between 9.5 and 10.5 million cells per ml of culture.
   d. Starting culture pH - between 7.20 and 7.60
   e. Sendai virus content - between 100 and 200 HAU/ml of final culture
   f. Antibiotic content - Neomycin, 25 micrograms per ml of final culture.
   g. Incubation temperature - between 37° and 39°C.
   h. Incubation time - between 12 and 20 hours.
   i. Continuous stirring is required.
4. The optimum pH of the culture is 7.4 and the pH is adjusted to this value with acid or base, as necessary.
5. The interferon cell culture represents living cells in active metabolism. In carrying out their life functions, the cells produce acid protein metabolytes which cause a small decrease in pH.
6. Cell concentration affects the pH of the culture over the course of the incubation period. At a concentration of about 10 million cells per ml the pH of the culture remains relatively stable over an 18 hour period and exhibits only a very slight alkaline shift. Cell concentrations significantly less than 10 million cells per ml exhibit a significant alkaline pH shift over the culture period. Cell concentrations significantly greater than 10 million cells per ml exhibit a strong acid pH shift.
7. An incubation temperature of between 37° and 39°C is acceptable although a temperature of between 37° and 38°C is preferred. Temperature is an important aspect of the process and must be adequately controlled over the incubation period. Temperatures below 37°C retard and may abolish viral activity and suppress interferon production. Temperatures above 39°C can entirely destroy the inducer virus.
8. Continuous stirring is also important. If stirring is discontinued for even a short period of time the interferon yield may fall dramatically.
9. After incubation is completed, the resulting culture medium contains interferon produced by the leukocytes. The medium is cleared by centrifugation of the cells and particulate matter including cellular debris. The crude interferon is characterized before processing is continued.
10. The crude interferon is clarified and concentrated using suitable filtration techniques and final sterilization is carried out by passage of the filtered interferon through additional sterilizing filters.
11. At the time of termination of the cell culture the pH is checked to determine if the culture has been stable over the incubation period and for the presence of gross bacterial contamination. If gross bacterial contamination exists, the interferon is not suitable for use and the contaminated cultures is inactivated and discarded.
12. If the culture is not contaminated, the filtered crude interferon is assayed for anti-viral activity. The following criteria must be met in order to assure that the clarified crude interferon is suitable for further processing:
   a. The protein content must be equal to or less than about 10% of the initial culture medium protein content; and
   b. The clarified crude interferon must be free of inducer virus as demonstrated by hemagglutination.
13. The clarified crude interferon is concentrated to approximately 1/25 of its original volume by passage through an appropriate filter under pressure. The filtrate is discarded and the concentrated retentate is retained. The final concentrated crude interferon should be 1/25 of the original volume and have a protein content of 2.5 times the initial culture protein content (approximately 2.5 mg/ml). The crude concentrate may be used or further purified. It can be stored at -7°C for up to 7 years without significant loss of activity or at -20°C for up to 2 years. Average figures for the interferon content and specific activity of the concentrated crude preparation range between 500,000 and 1,000,000 units per ml at between 200,000 and 400,000 units per mg protein content.
14. The crude and/or concentrated crude interferon may be purified to a specific activity in excess of 10⁵, 10⁶ or 10⁷ units (representing different grades of interferon as determined by purity) per mg protein content by concentration and/or a modification of the Cantell procedure using KSCN precipitation, extraction with ethanol and differential precipitation at varying pH.
15. The final interferon product is then tested for sterility, potency, specific activity and pyrogenicity. It can be stored as described above.
16. Prior to use, the purified interferon product is subjected to dialysis in order to remove any KSCN residue and to adjust the vehicle and pH of the final solution. The human leukocyte interferon is then ready for storage or use in the topical composition prepared in accordance with the invention.

The final solution prepared in this manner contains 500,000 to 1,000,000 Units/ml at between 200,000 to 400,000 Units/mg protein content. An International Reference Unit of interferon is defined as the reciprocal of the dilution that provides a 50% cytopathic effect reduction. The cytopathic effect reduction is observed by dilutions of the interferon as compared to an International Reference Interferon Standard and is determined by assaying the final product using a microbiological technique that measures the dye uptake of cells.

Several assays for specific interferon activity are known. The method developed by Kahn et al (Interferon, Properties and Clinical Uses, Kahn A, Hill N and Doon G (Eds.) Dallas: Leland Fikes Foundation Press, pp. 529-39, 1979) is based on the uptake of neural red dye by viable cells that have not undergone the cytopathic effect (CPE) as described by Finter, Armstrong and Pidot. Four conditions are set up as follows:
1. A culture of control cells to which no virus is added. This represents 0% CPE or inversely 100% CPE reduction;
2. A culture of cells with virus to which no interferon inhibitor is added. This represents 100% CPE or inversely 0% CPE reduction;
3. Cell cultures containing virus and serial dilutions of an International Reference Interferon Standard representing the spectrum of interferon inhibition anti-viral activity from 100% CPE reduction to 0% CPE reduction; and
4. Cell cultures containing virus and serial dilutions of an interferon representing the spectrum of interferon inhibition anti-viral activity from 100% CPE reduction to 0% CPE reduction. The interferon under test is used for comparison with the reference.

The test cells are human U amnion cells and the effector virus is Vesicular Stomatitis virus. The Interferon Unit is defined as the reciprocal of the dilution that will effect a 50% CPE reduction in the test culture.

The cultures are washed and saturated with neural red dye. Then the cultures are washed again to remove excess dye and the optical density is measured at 540 nm. The extraction fluid derived from 100% CPE reduction control is used as a standard as is the extraction fluid from the 0% CPE reduction control. The optical density obtained from reading the extraction fluid of the various reference and non-reference interferon dilutions can then be evaluated for the CPE percent reduction.

A simplistic analysis can then be formulated by plotting the optical density of the reference against the dilutions on graph paper. On the same graph, the ordinates of the non-reference interferon are plotted. At the point of intersection with the 50% CPE reduction limit, the reference and non-reference have the same value. The concentration is the ratio of their respective dilutions at that point. Since the value of the reference is known, the value of the sample under test can be calculated by interpolation.

The CPE percent reduction value of the optical density is converted to probits using a probit table and the probits are plotted on graph paper against the dilution value converted to the natural log in order to effect a linear regression. The Xʹ of Yʹ equal to 5.0 (50% CPE) is determined and the natural anti-log of this value represents the interferon titer. The determined assay will vary from day to day and a correction factor must be applied to all values taken from the graph equivalent to the ratio of the known value to the determined value. However, within a run there is little variation and the procedure is generally considered to be reliable.

The human leukocyte interferon topical composition is prepared by dispersing the interferon in a vehicle so that the interferon is present in a concentration of between 50,000 and 150,000 Units per gram of formulation. The preferred composition may also include up to about 15 weight percent of an enhancing penetrant, such as DMSO or low molecular weight dextran. In a preferred composition, up to 0.5% by weight of a softening agent, such as carboxymethyl cellulose, is added. It is most preferred that 5% by weight of DMSO be used as human tests indicate that there is no effect from the DMSO at this concentration as it penetrates. A low molecular weight dextran of 4,000 daltons can be substituted for the DMSO. When the dextran is utilized, the same percent by weight is included.

The softening agent can be used in an amount up to 0.5% by weight and, more preferably, between 0.05 and 0.3% by weight of the composition. In a most preferred embodiment of the invention, 0.1% by weight of carboxymethyl cellulose is used as a softening agent.

The vehicle used to prepare the composition is not critical. The vehicle may be any cosmetically acceptable vehicle that does not denature the interferon and is non-toxic. It has been found that unscented moisturizing formulas of the type generally used for dry skin care are suitable. Such moisturizing formulas generally are water and oil emulsions in which the human leukocyte interferon can be dispersed. Preferably, the vehicle is a lyophile non-toxic base. One such vehicle used in the following case studies is a Eucerin moisturizing formula obtained from Beiersdorf, Inc. of South Norwalk, Connecticut. The Eucerin moisturizing formula is a water in oil emulsion and is identified as containing water, petrolatum, mineral oil, mineral wax, wool wax, alcohol and 2-bromo-2-nitropropane 1,3 diol. In an especially preferred embodiment of the invention, the following formulation is used (all percentages are by weight):
Eucerin cream - 84%
Human leukocyte interferon (500,000 units per ml) - 10%
dimethyl sulfoxide (DMSO) - 5%
benzyl alcohol - 0.9%
carboxymethyl cellulose - 0.1%
During treatment with the human leukocyte interferon topical composition having 100,000 units/g, the composition is applied to the infected area about 4 times per day. A 10 gram sample is sufficient for 7 to 10 days of treatment. Thus, a patient applies about 1,000,000 units of interferon over the course of a 10 day treatment period.

### Case Studies

The following case studies demonstrate the positive results obtained by utilizing the human leukocyte interferon composition prepared in accordance with the invention. The human leukocyte interferon used was prepared by scale-up techniques of the procedure outlined above. The composition used for the case studies used human leukocyte interferon dispersed in a Eucerin base with 5% DMSO and 0.1% carboxymethyl cellulose, as set out above as a preferred embodiment. In each of these case studies, the patients were followed for a period of about 3 months, although the topical composition was applied for only the initial 7 to 10 days. The studies are set forth as illustrative and not in a limiting sense.

### Case 1

Male patient A had genital Herpes Simples outbreaks for about eleven years on a monthly basis. The ointment was applied to the infected area on the basis of four times per day. The usual duration of the lesions was seven days and the duration of pain was three days. With the application of the medication mild pain was experienced for only 24 hours and the lesions were healed within 72 hours. The patient did not experience a recurrence within 60 days.

### Case 2

Male patient B had experienced genital Herpes Simplex episodes for 10 years on a monthly basis. The usual duration periods of the outbreaks was between about 6 and 9 days with severe pain during the formation of the initial lesions. The ointment was applied to the affected area at the rate of four times per day. The application of the medication eliminated the pain within 24 hours and the time for healing was reduced to 72 hours. Patient did not experience a recurrence within 90 days.

### Case 3

Male patient C had experienced Genital Herpes Simplex for nine years with outbreaks on a monthly basis. The usual period of duration of the outbreak was five days and the duration of pain was two days. The ointment was applied four times per day onto the lesions. The patient experienced reduction of pain to only 24 hours and complete healing within 72 hours. The patient experienced recurrence at 53 days.

### Case 4

Male patient D had experienced genital Herpes Simplex for six years with 10 - 12 outbreaks per year. The usual duration of the outbreaks lasted for nine days with "on and off" pain for two days. The interferon ointment was applied to the infected areas at the rate of four times per day. The patient experienced a less severe episode than usual with increased healing time of four days and no pain within the first 24 hours use of the ointment. Recurrence occurred at 45 days.

### Case 5

Male patient E had suffered monthly with genital Herpes Simplex for two years with 10 day duration of the episodes and three days of significant pain and pruritus. The interferon ointment was applied to the lesions four times per day. The first noted improvement was the relief of pruritus and pain within 24 hours. The healing time was reduced from 10 days to 5 days. No recurrence occurred within 60 days.

### Case 6

Female patient F experienced monthly genital Herpes Simplex episodes. The usual duration of each episode being eight days with associated pain for two days. The patient was treated and applied interferon ointment four times per day to the infected areas. The patient experienced reduced pain with the ointment and a shortened duration period to five days with complete healing of the lesions. No recurrenc occurred within 60 days.

### Case 7

Female patient G had been infected with genital Herpes Simplex for five years. The patient experienced monthly herpetic episodes of a seven day duration with three days of pain. Interferon ointment was applied four times per day. Within 24 hours all pain and irritation had ceased and the healing time improved and shortened to five days. The patient did not experience a recurrence within 75 days.

Accordingly, a topical composition containing human leukocyte interferon in a gentle vehicle is provided. The topical composition also contains a penetrant such as DMSO or low molecular weight dextran and, preferably, a softening agent such as carboxymethyl cellulose. The topical composition prepared in accordance with the invention is useful for treatment of herpes simplex, venereal warts and condyloma.

## Claims

1. A topical composition for dermatological treatment comprising an- ointment-type vehicle, including at least an effective amount of human leukocyte interferon dispersed therein and present in an amount of up to 150,000 International Reference Units of Interferon Per Gram of composition, and from an effective amount up to 15 weight per cent of an enhancing penetrant based on the total weight of the composition.

2. The topical composition of claim 1, further including an effective amount of a softening agent.

3. The topical composition of claim 2, wherein the softening agent is carboxymethyl cellulose.

4. A topical composition for dermatological treatment comprising a lyophilic non-toxic base, human leukocyte interferon dispersed therein and present in an amount up to 150,000 International Reference Units of Interferon Per Gram of composition, up to 5% by weight of dimethyl sulfoxide and up to 0.5% by weight by carboxymethyl cellulose.

5. A method of improving the cosmetic appearance of the skin comprising applying to the skin a topical composition according to any one of claims 1 to 4.

6. Use as a cosmetic product of the topical composition in accordance with any one of claims 1 to 4.

## Patentansprüche

1. Topische Zusammensetzung zur dermatologischen Behandlung, umfassend ein ointment-artiges Vehikel, das mindestens eine wirksame Menge menschliches Leukozyten-Interferon dispergiert enthält, und das darin in einer Menge von bis zu 150 000 Internationalen Referenzeinheiten von Interferon pro Gramm Zusammensetzung vorliegt, und, bezogen auf das Gesamtgewicht der Zusammensetzung, eine wirksame Menge von bis zu 15 Gew.-% eines Penetrationsverstärkers.

2. Topische Zusammensetzung nach Anspruch 1, die darüber hinaus eine wirksame Menge eines Weichmachers enthält.

3. Topische Zusammensetzung nach Anspruch 2, worin der Weichmacher Carboxymethylcellulose ist.

4. Topische Zusammensetzung zur dermatologischen Behandlung, umfassend eine lyophile, nichttoxische Basis, darin dispergiertes menschliches Leukozyton-Interferon, das in einer Menge bis zu 150 000 Internationalen Referenzeinheiten von Interferon pro Gramm der Zusammensetzung vorliegt, bis zu 5 Gew.-% Dimethylsulfoxid und bis zu 0,5 Gew.-% Carboxymethylcellulose.

5. Verfahren zum Verbessern des kosmetischen Aussehens der Haut, umfassend das Anwenden einer topischen Zusammensetzung nach einem der Ansprüche 1 bis 4 auf die Haut.

6. Verwendung der topischen Zusammensetzung gemäß einem der Ansprüche 1 bis 4 als kosmetisches Produkt.

## Revendications

1. Composition topique pour traitement dermatologique comportant un véhicule du type pommade qui comprend au moins une quantité efficace d'interféron leucocytaire humain qui s'y trouve dispersé et est présent dans une quantité pouvant atteindre 150000 unités de référence internationale d'interféron par gramme de composition et à partir d'une quantité efficace pouvant atteindre 15% en poids d'un pénétrant favorisant basé sur le poids total de la composition.

2. Composition topique selon la revendication 1, comportant en outre une quantité efficace d'un agent adoucissant.

3. Composition topique selon la revendication 2, dans laquelle l'agent adoucissant est la cellulose carboxyméthyle.

4. Composition topique pour traitement dermatologique comportant une base lyophilique non toxique, de l'interféron leucocytaire humain dispersé dans celle-ci et présent dans une quantité pouvant atteindre 150000 unités de référence internationale d'interféron par gramme de composition et pouvant atteindre 5% en poids de diméthylsulfoxyde et jusqu'à 0,5% en poids de cellulose de carboxyméthyle.

5. Procédé pour améliorer la présentation cosmétique de la peau comportant l'application sur la peau d'une composition topique selon l'une quelconque des revendications 1 à 4.

6. Utilisation en tant que produit cosmétique de la composition topique selon l'une quelconque des revendications 1 à 4.
